# EUROPEAN PATENT APPLICATION

(11) **EP 3 900 572 A1**
(43) Date of publication of application: **27.10.2021**
(21) Application number: 21169202.5
(22) Date of filing: 19.04.2021
(51) Int. Cl.: A45D 24/00, A61H 7/00, A61H 23/02, A61N 1/04, A61N 1/36, A61N 5/06

(54) **MASSAGE PIN FOR SKIN BLOOD CIRCULATION PROMOTOR AND COUPLING METHOD THEREOF**

(30) Priority: 20.04.2020 KR 20200047693
(71) Applicant: Mooyoo Co., Ltd, Bundang-gu, Seongnam-si, Gyeonggi-do 13511 (KR); G.M Corporation Co., Ltd., Kita-ku Osaka-shi, Osaka 531-0072 (JP)
(72) Inventor: KIM, Mi-Ja, 17768 Gyeonggi-do (KR)
(74) Representative: Kohler Schmid Möbus Patentanwälte

(57) **Abstract**

The present invention relates to a massage pin for skin blood circulation promoters. More particularly, the present invention relates to a massage pin for skin blood circulation promoters, which has a lower end to/from which a silver conductor having stable electrical resistance is coupled or separated by a user of a skin blood circulation promoter, as needed. The massage pin according to the present invention includes: a coupling portion (171) formed at an upper end of the massage pin and press-fitted into a circuit board (130) disposed between a main body (110) and a cover (120) of a blood circulation promoter (100), the coupling portion (171) being formed of a conductive rubber; and an exposed portion (172) extending from a lower end of the coupling portion (171) and exposed outside through a massage pin-passage hole (122) formed through the cover (120), the exposed portion (172) being formed of a conductive rubber, wherein the exposed portion (172) has a downwardly tapered connection portion (173) extending from a lower end thereof and including a silver conductor (174) detachably coupled to the connection portion (173), the conductor (174) has a reception portion (174a) open at an upper portion thereof, an inner diameter of the reception portion (174a) of the conductor (174) is smaller than an outer diameter of a middle portion of the connection portion (173), and an outer diameter of the connection portion (173) is smaller than an outer diameter of the exposed portion (172).

## Description

### BACKGROUND OF THE INVENTION

### Technical Field

The present invention relates to a massage pin for skin blood circulation promoters. More particularly, the present invention relates to a massage pin for skin blood circulation promoters, which has a lower end to/from which a silver conductor having stable electrical resistance is coupled or separated by a user of a skin blood circulation promoter, as needed.

### Background Art

In general, human hair is a tissue composed of fibrous proteins called "keratin" and formed in the hair follicle, which is a sac-like structure about 4 mm under the skin, and consists of three layers.

An outermost layer is a hard, thin, scaly, colorless protective layer; a middle layer is the thickest of all three layers, is structured to support the hair, and determines color of the hair and whether the hair is straight or curly; and an innermost layer is composed of living cells and supplies nutrients for growth of the hair.

Growth of the hair follicle is influenced by male hormones. Hair in the armpits and the pubic region is sensitive to these hormones and appears during puberty before other body hairs appear, whereas hair on the head is influenced by dihydrotestosterone (DHT), which is an androgen.

An average person normally loses 50 to 100 hairs a day under the influence of various factors such as surrounding environment, season, race, physiological factors, stress, and disease, which is called degenerative hair loss.

Severe degenerative hair loss leads to baldness, which affects a person's looks or appearance. Accordingly, most bald-headed people wear a wig, or, more actively, try to treat baldness by inhibiting hair loss and promoting hair growth.

In an effort to inhibit degenerative hair loss, various drugs for external application or for administration have been released onto the market. However, use of these drugs is accompanied by concerns of side effects due to allergic reaction of the human body to the drugs or due to misuse of the drugs. In addition, surgical hair transplantation is prevalent in recent years. However, such surgical hair transplantation has problems of psychological distress and cost burden associated with surgery and discomfort for a certain period of time after surgery.

Therefore, many people prefer an inexpensive and easy-to-implement scalp massage method, that is, a method of promoting hair growth by tapping the scalp to provide an external physical stimulus to the dermal papilla of the hair follicle. Various scalp massagers are available as related products.

A conventional scalp massager has a structure in which a head portion having multiple brush pins and a grip portion are integrally coupled with an elastically deformable connector, wherein a user can obtain an effect of massaging their scalp by tapping the scalp with the head portion while holding the grip portion with their hand such that the brush pins of the head portion provide stimulus to the scalp.

However, since such a conventional scalp massager is configured such that the brush pins strike the scalp without all of them contacting the scalp, the impact can be concentrated only on a portion of the scalp, causing pain at the scalp and, at worst, damage to the scalp.

In order to solve such a problem, there has been proposed a method of exposing the scalp to laser beams using a stationary laser device. However, this method has problems in that it takes a long time to achieve desired effects due to hindrance of light transmission caused by hairs and in that, in the case of thick but fine (due to, for example, lack of nutrients) hair, it is difficult to deliver laser beams to the scalp.

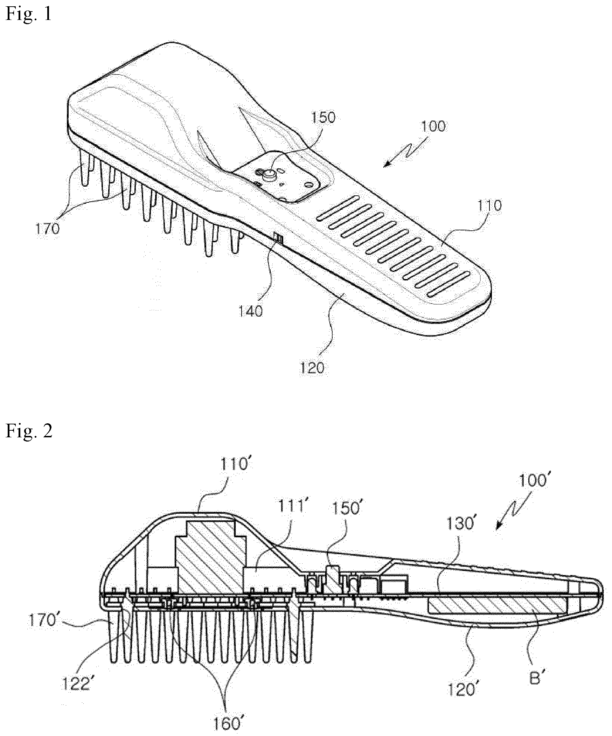

In order to solve this problem, there has been developed a skin blood circulation promoter (100') as shown in Figs. 1 and 2, which includes: a main body (110') formed with a recessed reception portion (111') with an open bottom; a cover (120') coupled to a lower portion of the main body (110') to close the reception portion (111'); a circuit board (130') securely mounted in the reception portion (111') of the main body (110'); a main switch (140') and an operation switch (150') each operably mounted on the circuit board (130'); a light emitting diode (160') powered by a battery (B') according to operation of the main switch (140') and the operation switch (150') and emitting a laser beam having a wavelength of 655 nm to 780 nm; and massage pins (170') formed of conductive rubber to transmit electric current from the battery (B') therethrough. In the skin blood circulation promoter (100'), when the operation switch (140') is operated with the main switch (150') in the "On" position, electric current is delivered from the battery (B') to the skin through the massage pins (170'), whereby the skin vibrates due to the electric current delivered through the massage pins (170'). In addition, the light emitting diode (160') emits a laser beam having a wavelength of 655 nm to 780 nm when powered by the battery (B'), thereby increasing blood flow through the skin, such as the scalp, while promoting cell metabolism. As a result, tiny blood-carrying capillaries and melanocytes can be activated, thereby inhibiting appearance of gray hairs and hair loss.

However, this skin blood circulation promoter has a problem in that, when the lower end of the massage (170') directly contacting the skin is worn out, electric current from the battery (B') is not properly delivered to the skin, such as the scalp, and thus there is no choice but to replace the skin blood circulation promoter with a new one.

### [Prior Art Documents]

### [Patent Documents]

(Patent Document 0001) Korean Patent Registration No. 10-0889464 (Registration date: March 11, 2009)

### SUMMARY OF THE INVENTION

### Technical Problems to be solved by the Invention

The present invention have been conceived to solve such problems in the art and it is an object of the present invention to provide a massage pin for skin blood circulation promoters, which is designed to cause skin, such as the scalp, to vibrate by applying electrical stimulation to the skin, the massage pin having a lower end to/from which a conductor directly contacting the skin is coupled/separated as needed by a user of a skin blood circulation promoter, whereby, when the conductor is worn out or contaminated and does not function properly due to contact with the skin while using the skin blood circulation promoter, the skin blood circulation promoter can be put back into use through a process in which the used conductor is separated from the massage pin, followed by coupling a new conductor to the lower end of the massage pin.

### Means to solve the problems

In accordance with an aspect of the present invention, there is provided a massage pin for skin blood circulation promoters. As in the art, the massage pin according to the present invention includes: a coupling portion (171) formed at an upper end of the massage pin and press-fitted into a circuit board (130) disposed between a main body (110) and a cover (120) of a blood circulation promoter (100), the coupling portion (171) being formed of a conductive rubber; and an exposed portion (172) extending from a lower end of the coupling portion (171) and exposed outside through a massage pin-passage hole (122) formed through the cover (120), the exposed portion (172) being formed of a conductive rubber.

In the massage pin according to the present invention, the exposed portion (172) has a downwardly tapered connection portion (173) extending from a lower end of the exposed portion (172) and including a conductor (174) detachably coupled to the connection portion (173), the conductor (174) being formed of silver, which has stable electrical resistance. In addition, the conductor (174) has a reception portion (174a) open at an upper portion thereof. Further, an inner diameter of the reception portion (174a) of the conductor (174) is smaller than an outer diameter of a middle portion of the connection portion (173) and an outer diameter of the connection portion (173) is smaller than an outer diameter of the exposed portion (172).

The connection portion (173) is formed of an elastically deformable conductive rubber and the reception portion (174a) has a depth greater than the length of the connection portion 173. Accordingly, when the conductor (174) is pushed toward the exposed portion (172) with the connection portion (173) received in the reception portion (174a), an outer wall of the connection portion (173) is brought into contact with an inner wall of the reception portion (174a) and is elastically deformed toward a center of the reception portion (174a), thereby allowing the conductor (174) to be securely held against the connection portion (173) while allowing a chamber (180) to be defined between a lower portion of the reception portion (174a) of the conductor (174) and the lower end of the connection portion (173). As a result, when the conductor (174) is exposed to impact upon contacting the skin, impact energy applied to the conductor 174 can be effectively absorbed by the chamber (180) and can be dispersed widely through the exposed portion (172), thereby preventing deformation of and damage to the connection portion (173) due to the impact applied to the conductor (174).

### Effects of the Invention

As described above, with the structure wherein the conductor is detachably coupled to the connection portion extending from a lower end of the exposed portion of the massage pin, the present invention can allow the conductor to be coupled to or separated from the connection portion as needed by a user of the skin blood circulation promoter, whereby, when the conductor is worn out or contaminated and does not function properly due to contact with the skin in use of the skin blood circulation promoter, the skin blood circulation promoter can be put back into use through a process in which the used conductor is separated from the connection portion of the massage pin, followed by coupling a new conductor to the connection portion thereof.

### Brief Description of Drawings

Fig. 1 is a perspective view of a typical skin blood circulation promoter.
Fig. 2 is a sectional view of a typical massage pin in use.
Fig. 3 is a sectional view showing placement of a massage pin for skin blood circulation promoters according to the present invention.
Fig. 4 is a perspective view of the massage pin for skin blood circulation promoters according to the present invention.
Fig. 5 is a sectional view of the massage pin for skin blood circulation promoters according to the present invention.
Fig. 6a is an enlarged sectional view of the massage pin for skin blood circulation promoters according to the present invention, with a conductor separated from a connection portion of the massage pin.
Fig. 6b is an enlarged sectional view of the massage pin for the skin blood circulation promoter according to the present invention, wherein the conductor is in the process of being coupled to the connection portion of the massage pin.
Fig. 6c is an enlarged view of the massage pin for the skin blood circulation promoter according to the present invention, with the conductor securely coupled to the connection portion of the massage pin.

### Detailed Description of the Invention

Embodiments of the invention will now be described in detail with reference to the accompanying drawings.

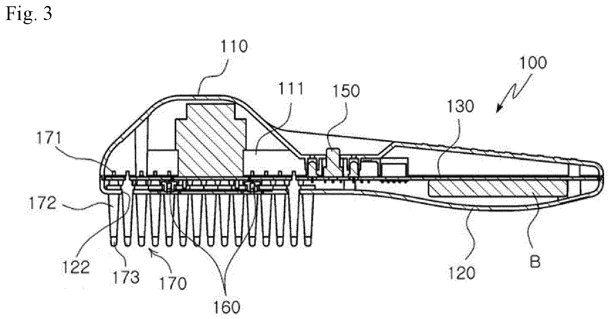

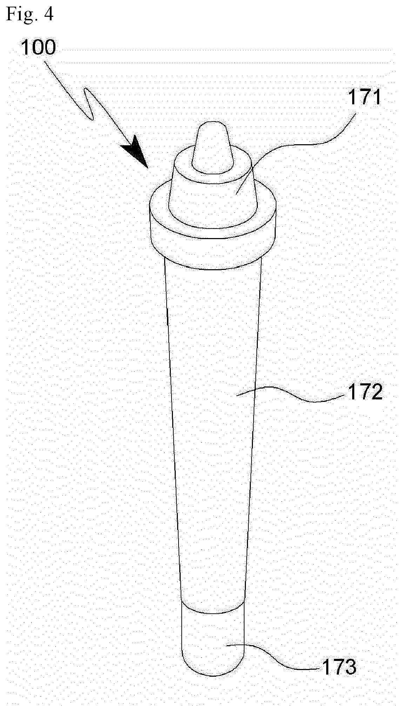

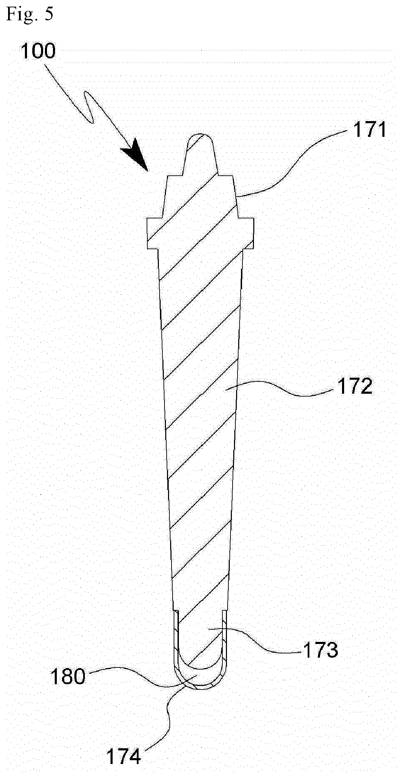

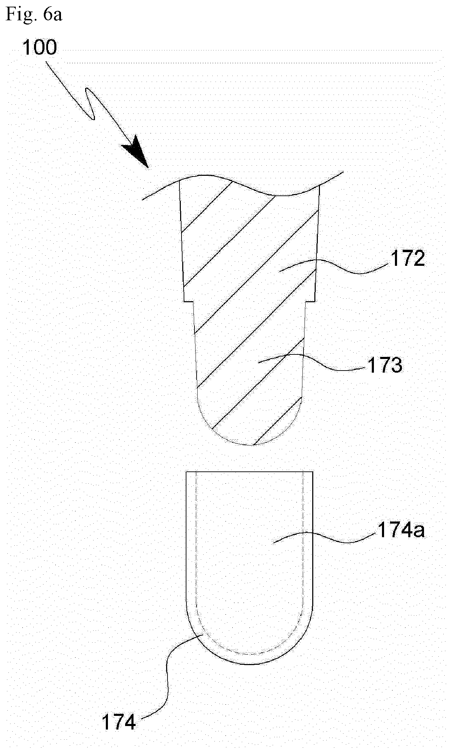

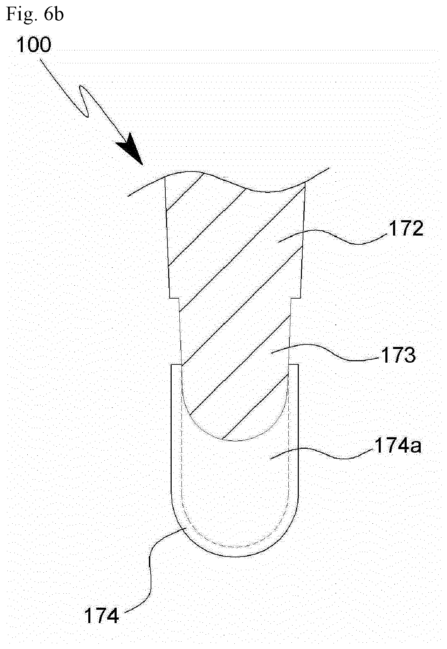

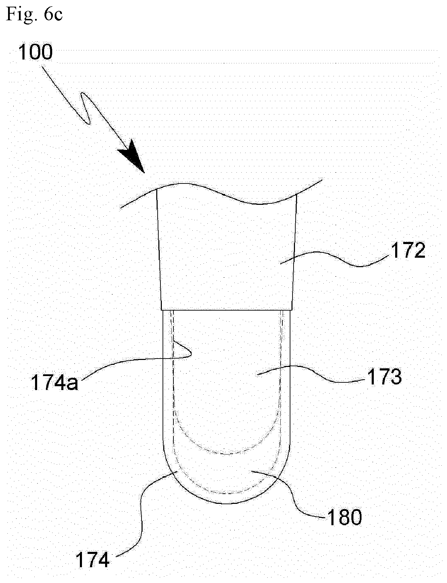

Fig. 3 is a sectional view showing placement of a massage pin for a skin blood circulation promoter according to the present invention, Fig. 4 is a perspective view of the massage pin for the skin blood circulation promoter according to the present invention, Fig. 5 is a sectional view of the massage pin for the skin blood circulation promoter according to the present invention, Fig. 6a is an enlarged sectional view of the massage pin for the skin blood circulation promoter according to the present invention, with a conductor separated from a connection portion of the massage pin, Fig. 6b is an enlarged sectional view of the massage pin for the skin blood circulation promoter according to the present invention, wherein the conductor is in the process of being coupled to the connection portion of the massage pin, and Fig. 6c is an enlarged view of the massage pin for the skin blood circulation promoter according to the present invention, with the conductor securely coupled to the connection portion of the massage pin.

Referring to Fig. 3, a massage pin for skin blood circulation promoters according to the present invention includes: a coupling portion (171) formed at an upper end of the massage pin and press-fitted into a circuit board (130) disposed between a main body (110) and a cover (120) of a blood circulation promoter (100), the coupling portion being formed of a conductive rubber; and an exposed portion (172) extending from a lower end of the coupling portion (171) and exposed outside through a massage pin-passage hole (122) formed through the cover (120).

In addition, the exposed portion (172) has a downwardly tapered connection portion (173) extending from a lower end of the exposed portion (172) and including a conductor (174) detachably coupled to the connection portion (173), the conductor (174) being formed of silver, which has stable electrical resistance.

Further, the conductor (174) has a reception portion (174a) open at an upper portion thereof, wherein an inner diameter of the reception portion (174a) is smaller than an outer diameter of a middle portion of the connection portion (173) and an outer diameter of the connection portion (173) is smaller than an outer diameter of the exposed portion (172).

The connection portion (173) is formed of an elastically deformable conductive rubber, and a depth of the reception portion (174a) is greater than a length of the connection portion (173).

According to the present invention, first, the connection portion (173) is inserted into the reception portion (174a) of the conductor (174) positioned such that a lower end of the connection portion (173) is aligned with a center of the reception portion (174a), as shown in FIG. 6.

Then, the connection portion (173) is brought into contact with an inner surface of the reception portion (174a) such that the conductor (174) is temporarily held against the middle portion of the connection portion (173).

Then, the conductor (174) temporarily held against the middle portion of the connection portion (173) is moved upwardly of the connection portion (173) such that an outer surface of the connection portion (173) is elastically deformed, by elastic force thereof, against an inner wall of the reception portion (174a), the inner diameter of which is smaller than the outer diameter of the connection portion (173), to allow the connection portion (173) to be received in the reception portion (174a). After the connection unit (173) is elastically deformed and received in the reception portion (174a), the conductor (174) is securely held against the connection portion (173) by elastic force thereof.

As described above, the depth of the receiving portion (174a) is greater than the length of the connection portion (173). Accordingly, when the conductor (174) is pushed toward the exposed portion (172) with the connection portion (173) received in the reception portion (174a), a chamber (180) is defined between a lower portion of the reception portion (174a) of the conductor (174) and the lower end of the connection portion (173). As a result, when the conductor (174) is exposed to impact upon contacting the skin, impact energy applied to the conductor (174) can be effectively absorbed by the chamber (180) and can be dispersed widely through the exposed portion (172).

After coupling the conductor (174) to the connection portion (173) as described above, the coupling portion (171) formed of the conductive rubber is fitted into the circuit board (130) disposed between the main body (110) and the cover (120) such that the massage pin (170) with the conductor (174) coupled to the connection portion (173) is secured to the circuit board (130), followed by exposing the exposed portion (172) outside by allowing the exposed portion (172) to pass through the massage pin-passage hole (122) formed through the cover (120).

Accordingly, when an operation switch (not shown) is operated with a main switch (150) in the "On" position, electric current is delivered from a battery (B) to the connection portion (173) of the massage pin (170) and then delivered to the skin through the conductor (174). As a result, the skin vibrates due to the electric current delivered through the conductor 174, thereby increasing blood flow through the skin, such as the scalp, while promoting cell metabolism. As a result, tiny blood-carrying capillaries can be activated, thereby inhibiting appearance of gray hairs and hair loss.

When the conductor (174) of the massage pin (170) is deformed or contaminated due to contact with the skin while using the blood circulation promoter (100), the conductor (174) is pulled downwardly of the connection portion (173) to be separated from the connection portion (173). Then, a new conductor (174) is coupled to the connection portion (173) with the used conductor (174) removed therefrom, followed by putting the skin blood circulation promoter (100) back into use.

### <Reference Numerals for the Drawings>

170: Massage pin
171: Coupling portion
172: Exposed portion
170: Connection portion
174: Conductor
174a: Reception portion
180: Chamber

## Claims

1. A massage pin for skin blood circulation promotors, comprising:
a coupling portion (171) formed at an upper end of the massage pin and press-fitted into a circuit board (130) disposed between a main body (110) and a cover (120) of a blood circulation promoter (100), the coupling portion (171) being formed of a conductive rubber; and
an exposed portion (172) extending from a lower end of the coupling portion (171) and exposed outside through a massage pin-passage hole (122) formed through the cover (120), the exposed portion (172) being formed of a conductive rubber,
wherein the exposed portion (172) has a downwardly tapered connection portion (173) extending from a lower end thereof and including a silver conductor (174) detachably coupled to the connection portion (173), the silver conductor (174) having a reception portion (174a) open at an upper portion thereof,
an inner diameter of the reception portion (174a) of the conductor (174) is smaller than an outer diameter of a middle portion of the connection portion (173),
an outer diameter of the connection portion (173) is smaller than an outer diameter of the exposed portion (172),
the connection portion (173) is formed of an elastically deformable conductive rubber,
a depth of the reception portion (174a) is greater than a length of the connection portion (173), and
a chamber (180) is defined between a lower portion of the reception portion (174a) of the conductor (174) and a lower end of the connection portion (173).

2. A coupling method of a massage pin for skin blood circulation promoters, the method sequentially comprising the steps of:
inserting a connection portion (173) into a reception portion (174a) of a conductor (174) positioned such that a center of the reception portion (174a) is aligned with a lower end of the connection portion (173);
bringing the connection portion (173) into contact with an inner surface of the reception portion (174a) such that a conductor (174) is temporarily held against a middle portion of the connection portion (173);
moving the conductor (174) temporarily held against the middle portion of the connection portion (173) upwardly of the connection portion (173);
allowing an outer surface of the connecting portion (173) to be elastically deformed, by elastic force thereof, against an inner wall of the reception portion (174a), an inner diameter of which is smaller than an outer diameter of the connection portion (173), such that the connecting portion (173) is received in the reception portion (174a);
allowing the conductor (174) to be securely held, by elastic force thereof, against the connection portion (173) elastically deformed and received in the reception portion (174a);
defining a chamber (180) between a lower portion of the reception portion (174a) of the conductor (174) and a lower end of the connecting portion (173) by pushing the conductor (174) toward the exposed portion (172) with the connection portion (173) received in the reception portion (174a);
fitting a coupling portion (171) formed of a conductive rubber into a circuit board (130) disposed between a main body (110) and a cover (120) of a skin blood circulation promoter (100) such that the massage pin (170) with the conductor (174) coupled to the connection portion (173) is secured to the circuit board (130);
exposing an exposed portion (172) outside by allowing the exposed portion (172) to pass through a massage pin-passage hole (122) formed through the cover (120);
separating the conductor (174) from the connection portion (173) by pulling the conductor (174) downwardly of the connection portion (173) when the conductor (174) is deformed or contaminated due to contact with skin while using the skin blood circulation promoter (100); and
coupling a new conductor (174) to the connection portion (173) with the conductor (174) separated therefrom, followed by putting the skin blood circulation promoter (100) back into use.
